# EUROPEAN PATENT APPLICATION

(11) **EP 4 239 075 A1**
(43) Date of publication of application: **06.09.2023**
(21) Application number: 22160288.1
(22) Date of filing: 04.03.2022
(51) Int. Cl.: C12P 19/14, C12P 19/22, C12P 7/06, C12G 3/021, C12G 3/022

(54) **PROCESS FOR THE PRODUCTION OF ETHANOL AND/OR FERMENTATION BY-PRODUCTS FROM A STARCH-CONTAINING BIOMASS**

(71) Applicant: Technische Universität München, 80333 München (DE)
(72) Inventor: MAREK, Manuel, Freising (DE); VIEBAHN, Lukas, Freising (DE); KUPETZ, Michael, Freising (DE); MINCEVA, Mirjana, Freising (DE)
(74) Representative: Maiwald GmbH

(57) **Abstract**

The present invention relates to a process for the production of ethanol and/or fermentation by-products from a starch-containing biomass as well as a spirit prepared by the process for the production of ethanol and/or fermentation by-products from starch-containing biomass.

## Description

The present invention relates to a process for the production of ethanol and/or fermentation by-products from a starch-containing biomass as well as a spirit prepared by the process for the production of ethanol and/or fermentation by-products from starch-containing biomass.

Mashing processes are well known in the art and are used to break down the starch contained in a raw material into water-soluble saccharides. As this process is massively accelerated by heat-induced swelling of the starch granules (gelatinization), all common processes aim to heat the water-starch suspension. After this, enzymes (amylases) added to the suspension can break down the starch, which is then easily accessible, into fermentable sugars within 15 minutes. However, the enzymes contained in the raw material are relatively quickly deactivated by the high temperatures (> 60°C) used in the process and are not further usable in the further process. Malted grains are particularly suitable for this process due to the formation and release of endogenous enzymes. However, many unmalted raw materials (rye, barley, wheat, rice, corn and the like) are also used for the production of liquids. They are usually treated in a so-called cereal cooker before the enzymes can attack the starch because of the very high gelatinization temperatures of in some cases >90°C.

In contrast to beer, in the production of spirits the insoluble grain components do not necessarily have to be separated from the liquid because a separation of the solid and liquid components takes place in the following distillation. In some regions, solids such as husks are left in the fermenting mash (grain-in). In principle, little has changed in this technology over the last hundred years. Optimum temperatures for gelatinization of the respective raw materials and enzymatic degradation processes were determined and the process optimized accordingly. Hot starch digestion is the most widely used and applied method internationally.

Nevertheless, various processes have been developed and presented in the past decades. For example, DE 3 638 529 C2 discloses the Hohenheim dispersing mashing process describing a mashing process using a disperser for ultra-fine grinding. The process is based on a classical heating of the mash to achieve optimal saccharification temperatures and subsequent cooling to pitching temperature. Enzymes are additionally added for liquefaction and saccharification, since the raw materials described are not limited to malt.

Furthermore, there are numerous patents and publications such as EP 0 295 358 A1, DD 267 508 A1 and DD 205 928A1 under the term "cold mashing process". However, they are referred to as cold only because the mash either swells cold and is subsequently saccharified warm, or is only heated to the saccharification temperature and then not cooked. Generally, these processes have in common a final heating to at least 40°C.

In biofuel production, e.g. from US 8,647,850 B2 and US 5,100,791 A, a process is known as "simultaneous saccharification and fermentation" (SSF). It describes a continuous saccharification by added enzymes during fermentation. These processes are often used for the digestion of cellulose with the aid of special microorganisms. Applications in the beverage industry are not known.

UK patent application GB 2 316 951 A describes a fermentation of malt grist at room temperature. However, the use is limited to malt, without the addition of enzymes, without fine grinding, and at fermentation temperatures not exceeding 24°C.

However, the processes of the prior art provide a rather low efficiency as the water as well as energy consumptions are rather high.

In view of the above, there is still a need in the art for a process for the production of ethanol and/or fermentation by-products that is suitable for the production of spirits and especially a process that provides high efficiency in terms of energy and water consumption. Furthermore, there is a need for a process for the production of ethanol and/or fermentation by-products allowing short mash times, low costs for operating and investment as well as an improved footprint in distilleries compared to conventional mashing equipment.

Accordingly, it is an object of the present invention to provide a process for the production of ethanol and/or fermentation by-products. Furthermore, it is desirable that the process for the production of ethanol and/or fermentation by-products is suitable for the production of spirits. Furthermore, it is desirable that the process provides high efficiency in terms of energy and water consumption.

The foregoing and other objects are solved by the subject-matter as defined in the independent claims. Advantageous embodiments of the present invention are defined in the corresponding subclaims.

According to one aspect of the present invention, a process for the production of ethanol and/or fermentation by-products from a starch-containing biomass is provided, the process comprising the steps of
a) providing a starch-containing biomass,
b) providing yeast,
c) preparing a fermentation mixture by mixing the starch-containing biomass of step a) with water,
d) mechanical comminution of the starch-containing biomass in the fermentation mixture of step c) at a temperature of at most 45°C,
e) adding yeast before and/or during and/or after mechanical comminution step d),
f) incubating the fermentation mixture obtained in comminution step d),
g) distillation of the fermentation mixture obtained in step f) for purifying the prepared ethanol and/or fermentation by-products.

According to one embodiment, the starch-containing biomass is selected from the group comprising non-malted grain, non-milled grain, non-malted non-milled grain, non-milled malted grain and mixtures thereof or the starch-containing biomass is selected from the group comprising non-malted grain, non-milled grain, non-malted non-milled grain, non-milled malted grain and mixtures thereof in admixture with milled grain or milled malted grain.

According to another embodiment, the starch-containing biomass is selected from the group comprising barley, rye, wheat, corn, rice, potatoes, oats, buckwheat, sorghum, millet, einkorn, emmer, triticale and mixtures thereof.

According to yet another embodiment, the weight ratio of starch-containing biomass to water in step c) ranges from 1:0.3 to 1:5, preferably from 1:0.5 to 1:4, more preferably from 1:1 to 1:4 and most preferably from 1:2 to 1:3.5.

According to one embodiment, the water in step c) is tap water and/or pre-treated water.

According to another embodiment, the water in step c) has a temperature ranging from 1 to 25°C, preferably from 10 to 25°C.

According to yet another embodiment, the mechanical comminution of step d) is carried out by applying shear force, impact force, frictional force or cutting force.

According to one embodiment, mechanical comminution step d) is carried out by using a dispersing device.

According to another embodiment, in step e) the yeast is added after step d), preferably the yeast is added before saccharification of a maximum of 20% of the starch in the fermentation mixture has taken place.

According to yet another embodiment, the process further comprises a step e1) of adding starch- and/or sugar-hydrolyzing enzymes and/or enzymes for liquefaction during any one of steps c) to f).

According to one embodiment, incubating step f) is carried out at 10 to 45°C, preferably at 28 to 45°C, e.g. at about 30°C.

According to another embodiment, incubating step f) is carried out for 24 to 240 hours, preferably for 48 to 96 hours.

According to yet another embodiment, the process further comprises a step h) of aging the ethanol and/or fermentation by-products obtained in distillation step g).

According to one embodiment, the process is for the production of a spirit, preferably the spirit is selected from the group comprising whiskey, vodka, corn-brand, brandy, rice spirit and flavoured spirits.

According to another aspect of the present invention, a spirit prepared by the process for the production of ethanol and/or fermentation by-products from starch-containing biomass as defined herein, is provided. Preferably, the spirit is selected from the group comprising whiskey, vodka, corn-brand, brandy, rice spirit and flavoured spirits.

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

In the following, preferred embodiments of the inventive process will be set out in more detail. It is to be understood that these embodiments and details also apply to the inventive products described herein.

The process for the production of ethanol and/or fermentation by-products from a starch-containing biomass comprises the steps of
a) providing a starch-containing biomass,
b) providing yeast,
c) preparing a fermentation mixture by mixing the starch-containing biomass of step a) with water,
d) mechanical comminution of the starch-containing biomass in the fermentation mixture of step c) at a temperature of at most 45°C,
e) adding yeast before and/or during and/or after mechanical comminution step d),
f) incubating the fermentation mixture obtained in comminution step d),
g) distillation of the fermentation mixture obtained in step f) for purifying the prepared ethanol and/or fermentation by-products.

According to step a) of the inventive process, a starch-containing biomass is provided.

The starch-containing biomass of step a) may be any kind of material that may be used for the preparation of spirits. More precisely, the starch-containing biomass of step a) maybe any kind of material having a content of starch of at least 10 wt.-%, preferably at least 20 wt.-% more preferably at least 30 wt.-%, even more preferably at least 40 wt.-% and most preferably at least 50 wt.-%, based on the total weight of the starch-containing biomass of step a). For example, the starch-containing biomass of step a) maybe any kind of material having a content of starch ranging from 50 to 90 wt.-%, based on the total weight of the starch-containing biomass of step a).

Thus, the starch-containing biomass of step a) may be selected from grain, fruits, tuber, plants, vegetables, roots, seeds and mixtures thereof.

In one embodiment, it is preferred that the starch-containing biomass of step a) is selected from the group comprising non-malted grain, non-milled grain, non-malted non-milled grain, non-milled malted grain and mixtures thereof. Preferably, the starch-containing biomass of step a) is selected from non-malted grain and/or non-milled grain. Most preferably, the starch-containing biomass of step a) is selected from non-malted grain or non-milled grain.

In another embodiment, the starch-containing biomass of step a) is provided in admixture with milled grain or milled malted grain. Preferably, the starch-containing biomass is thus selected from the group comprising non-malted grain, non-milled grain, non-malted non-milled grain, non-milled malted grain and mixtures thereof in admixture with milled grain or milled malted grain.

The wording "admixture with" in the meaning of the present invention refers to a mixture in which the admixed milled grain or milled malted grain is present in minor amounts. That is to say, the admixed milled grain or milled malted grain is present in amounts ranging from 1 to 49 wt.-%, preferably from 2 to 40 wt.-%, more preferably from 3 to 30 wt.-% and most preferably from 4 to 25 wt.-%, based on the total weight of the starch-containing biomass of step a).

It is appreciated that the grain of the milled grain or milled malted grain and the one selected from the group comprising non-malted grain, non-milled grain, non-malted non-milled grain and non-milled malted grain may be the same or different. Preferably, the grain of the milled grain or milled malted grain and the one selected from the group comprising non-malted grain, non-milled grain, non-malted non-milled grain and non-milled malted grain are the same.

Such materials are well known in the art and the skilled person knows how to obtain such materials.

For example, the starch-containing biomass is selected from the group comprising barley, rye, wheat, corn, rice, potatoes, oats, buckwheat, sorghum, millet, einkorn, emmer, triticale and mixtures thereof. Preferably, the starch-containing biomass is selected from the group comprising barley, rye, wheat, corn, rice, oats, buckwheat, sorghum, millet, einkorn, emmer and triticale.

In one embodiment, the starch-containing biomass is barley.

It is appreciated that the starch-containing biomass may be a pre-treated starch-containing biomass. For example, the starch-containing biomass may be pre-treated by germination.

Additionally or alternatively, the starch-containing biomass may be pre-treated by gelatinization, e.g. of flaked corn, and/or mechanical starch degradation.

In general, such pre-treatment steps are well known in the art and the skilled person is well aware of conditions for such pre-treatment steps, which can be adapted according to the specific needs.

According to step b) of the inventive process, yeast is provided.

It is to be noted that any kind of yeast may be used in the inventive process that is known for the production of the spirits to be prepared.

In one embodiment, the yeast comprises species and/or subspecies selected from the group comprising Saccharomyces, Brettanomyces, Dekkera and mixtures thereof. It is further to be noted that the yeast may comprise hybrids of afore-mentioned yeasts, genetical modifications and/or mixtures of different strains of said yeasts. Preferably, the yeast comprises species and/or subspecies selected from the group comprising Saccharomyces cerevisiae, Saccharomyces pastorianus, Saccharomyces cerevisiae var. diastaticus and mixtures thereof. For example, the yeast may be distiller's yeast "Pinnacle MG+".

The yeast is preferably added in typical amounts to the fermentation mixture. The amount of yeast may depend on the yeast used in the process. However, the yeast is typically added in amounts ranging from 0.1 to 10 g/L of the fermentation mixture. For example, the yeast is added in amounts ranging from 0.5 to 8 g/L, preferably from 0.5 to 6 g/L and most preferably from 0.5 to 5 g/L, of the fermentation mixture. It is to be noted that it is also possible to use liquid yeast and/or harvested yeast. The liquid yeast and/or harvested yeast may be obtained from another process according to the invention. If liquid yeast and/or harvested yeast is used in the inventive process, the yeast is preferably added in rates of 1 million cells/ml to 40 million cells/ml, more preferably 5 million cells/ml to 25 million cells/ml.

According to step c) of the inventive process, a fermentation mixture is prepared by mixing the starch-containing biomass of step a) with water.

The water in step c) is preferably tap water and/or pre-treated water. For example, the water in step c) is tap water and pre-treated water. Alternatively, the water in step c) is tap water or pre-treated water. Preferably, the water in step c) is pre-treated water. It is to be noted that the pre-treatment of the water may include an adjustment of pH, e.g. to a pH ranging from 4 to 6, filtration, e.g. using carbon filter, additions of salts, e.g. calcium sulphate, sodium bicarbonate, calcium chloride and/or magnesium sulphate, reverse osmosis and combinations thereof.

It is appreciated that the water in step c) preferably has a temperature not exceeding room temperature. Thus, the water in step c) preferably has a temperature ranging from 1 to 25°C. More preferably, the water in step c) has a temperature ranging from 10 to 25°C. For example, the water in step c) has a temperature ranging from 15 to 25°C.

In one embodiment of the present process, the water in step c) has a temperature of about 21 °C (± 2°C).

In order to obtain a suitable fermentation mixture for further processing in step c), it is preferred that the starch-containing biomass and water are provided in a specific weight ratio. In one embodiment, the weight ratio of starch-containing biomass to water in step c) ranges from 1:0.3 to 1:5. Preferably, the weight ratio of starch-containing biomass to water in step c) ranges from 1:0.5 to 1:4, more preferably from 1:1 to 1:4 and most preferably from 1:2 to 1:3.5 .

According to step d) of the present process, the starch-containing biomass in the fermentation mixture of step c) is mechanically comminuted at a temperature of at most 45°C.

It is appreciated that the wording "mechanically comminuted" or "mechanical comminution" in the meaning of the present invention refers to a process step in which the starch-containing biomass in the water of the fermentation mixture of step c) is reduced in particles size and the starch-containing biomass and water in the fermentation mixture are homogenously mixed by the impact of a secondary body such as mechanical mixing and/or grinding means. That is to say, the mechanical comminution of the starch-containing biomass in the fermentation mixture is carried out in the presence of grinding devices, such as balls and/or blades, that are typically used for mixing and/or grinding particulate materials in a liquid medium.

Thus, any kind of mixing and/or grinding means suitable for wet mixing and/or grinding known in the art can be used in mechanical comminution step d). It is thus advantageous if the mechanical comminution of step d) is carried out by applying shear force, impact force, frictional force or cutting force. For example, the mechanical comminution of step d) is carried out by applying shear force.

In one embodiment, mechanical comminution step d) is preferably carried out in a dispersing device, a ball mill, a rod mill, a vertical bead mill, an attrition mill, a pin mill, a hammer mill, a pulveriser, a shredder, a roller mill, a hammer mill, or other such equipment known to the skilled person.

Preferably, mechanical comminution step d) is carried out by using a dispersing device.

If a dispersing device for mechanical comminution step d), it is preferred that the speed of the dispersing device is at least 4000 rpm, more preferably at least 5000 rpm and most preferably at least 6000 rpm. For example, the speed of the dispersing device ranges from 4000 to 15000 rpm, more preferably from 5000 to 9000 rpm and most preferably from 6000 to 8000 rpm.

Additionally or alternatively, the dispersing device for mechanical comminution step d) has a grinding gap ranging from 0.5 to 2 mm, preferably from 0.8 to 1.2 mm.

It is appreciated that mechanical comminution step d) is carried out at a temperature of at most 45°C. For example, mechanical comminution step d) is carried out at a temperature of at most 40°C and most preferably of at most 35°C. Preferably, mechanical comminution step d) is carried out at a temperature ranging from 1 to 45°C, more preferably from 1 to 40°C and most preferably from 1 to 35°C. More preferably, mechanical comminution step d) is carried out at a temperature ranging from 10 to 45°C, more preferably from 10 to 40°C and most preferably from 10 to 35°C. For example, mechanical comminution step d) is carried out at a temperature ranging from 15 to 35°C, more preferably from 15 to 40°C and most preferably from 15 to 35°C.

In this regard, it is to be noted that mechanical comminution step d) is stopped if the maximum temperature of 45°C, preferably of 40°C, more preferably of 35°C and most preferably of 32°C, is reached. Thus, there is no additional cooling in mechanical comminution step d) necessary or carried out resulting in a more efficient energy consumption of the overall process.

Mechanical comminution step d) may be carried out in batch or continuous mode, preferably in continuous mode.

According to step e) of the present process, yeast is added before and/or during and/or after mechanical comminution step d).

For example, the yeast is added before and during or after mechanical comminution step d). Alternatively, the yeast is added before or during and after mechanical comminution step d), preferably during and after mechanical comminution step d). Alternatively, the yeast is added before and during and after mechanical comminution step d). Alternatively, the yeast is added before or during or after mechanical comminution step d), preferably during or after mechanical comminution step d).

In one embodiment, the yeast is added after mechanical comminution step d).

As continuous saccharification of the starch in the fermentation takes place in the fermentation mixture during incubating, it is preferred that the yeast is added before complete saccharification of the starch in the fermentation mixture has taken place. Thus, it is preferred that the yeast is added before saccharification of a maximum of 20% of the starch in the fermentation mixture has taken place. More preferably, the yeast is added before saccharification of a maximum of 10% of the starch in the fermentation mixture has taken place.

Thus, the yeast is preferably added after mechanical comminution step d) and before incubating step f).

In one embodiment, the process further comprises a step e1) of adding starch- and/or sugar-hydrolyzing enzymes and/or enzymes for liquefaction to the fermentation mixture during any one of steps c) to f). Preferably, starch- and/or sugar-hydrolyzing enzymes and/or enzymes for liquefaction are added to the fermentation mixture during step c), step d) or step f). For example, starch- and/or sugar-hydrolyzing enzymes and/or enzymes for liquefaction are added to the fermentation mixture during step c), step d) or step f). Preferably, starch- and/or sugar-hydrolyzing enzymes and/or enzymes for liquefaction are added to the fermentation mixture during step c) or step d). More preferably, starch- and/or sugar-hydrolyzing enzymes and/or enzymes for liquefaction are added to the fermentation mixture after step d).

It is to be noted that any kind of starch- and/or sugar-hydrolyzing enzymes and/or enzymes for liquefaction may be used in the inventive process that is known for the production of the spirits to be prepared.

For example, the starch- and/or sugar-hydrolyzing enzymes and/or enzymes for liquefaction may be selected from glucosidases, amylases, pullulanases, beta-glucanases, cellulases, xylanases, arabinosidases, arabinofuranosidases, feruloyl esterases and mixtures thereof.

The starch- and/or sugar-hydrolyzing enzymes and/or enzymes for liquefaction is/are preferably added in typical amounts to the fermentation mixture. The amount of starch- and/or sugar-hydrolyzing enzymes and/or enzymes for liquefaction may depend on the enzyme used in the process and the raw materials used. However, the starch- and/or sugar-hydrolyzing enzymes and/or enzymes for liquefaction is/are typically added in amounts ranging from 0.1 to 10 g/kg of the starch-containing biomass. For example, the starch- and/or sugar-hydrolyzing enzymes and/or enzymes for liquefaction is/are added in amounts ranging from 0.1 to 8 g/kg, preferably from 0.1 to 6 g/kg and most preferably from 0.2 to 5 g/kg, of the starch-containing biomass.

According to step f) of the present process, the fermentation mixture obtained in comminution step d) is incubated.

As the mechanical comminution of step d) is carried out at a temperature of at most 45°C, it is appreciated that the fermentation mixture obtained in comminution step d) has a temperature of at most 45°C. Thus, the fermentation mixture obtained in comminution step d) can be directly subjected to incubating step f), i.e. without cooling the fermentation mixture obtained in comminution step d) before it is subjected to incubating step f). This is specifically advantageous because enzymes typically added to the fermentation mixture provide the most advantageous degradation rates at such a temperature and thus a continuous saccharification during the process can be provided.

Thus, it is specifically preferred that the temperature at the beginning and during incubating step f) is at most 45°C, preferably at most 40°C, more preferably at most 35°C and most preferably at most 32°C. For example, incubating step f) is carried out at a temperature ranging from 10 to 45°C, more preferably from 10 to 40°C and most preferably from 10 to 35°C. For example, incubating step f) is carried out at a temperature ranging from 28 to 45°C, more preferably from 28 to 40°C and most preferably from 28 to 35°C. In a preferred embodiment, incubating step f) is carried out at a temperature of about 30°C.

In order to ensure a complete saccharification, it is preferred that the fermentation mixture is incubated for a sufficient time. Thus, incubating step f) is preferably carried out for 24 to 240 hours. More preferably, incubating step f) is carried out for 48 to 96 hours.

According to step g) of the present process, the fermentation mixture obtained in step f) is distilled for purifying the prepared ethanol and/or fermentation by-products.

In general, distillation step g) can be carried out by any means known for distilling the products to be prepared by the present process. The skilled person is also aware of conditions for distilling, which can be adapted according to the specific needs. For example, distillation step g) can be carried out in continuous or batch mode, preferably continuous mode. Additionally or alternatively, distillation step g) can be carried out with rectification steps for further purification of the distilled spirit.

It is appreciated that purified ethanol and/or fermentation by-products are obtained in the distillation of step g). Preferably, ethanol or fermentation by-products are obtained in the distillation of step g). Alternatively, ethanol and fermentation by-products are obtained in the distillation of step g).

In one embodiment, the process further comprises a step h) of aging (also called maturating) the ethanol and/or fermentation by-products obtained in distillation step g).

In general, aging step h) can be carried out by any means known for aging the products to be prepared by the present process. The skilled person is also aware of conditions for aging, which can be adapted according to the specific needs. For example, aging step h) can be carried out by adding water in order to lower the alcohol level in the products to be prepared. Additionally or alternatively, aging step h) can be carried out by aging the products in barrels.

Such aging step h) may be for example carried out by storing the ethanol and/or fermentation by-products obtained in distillation step g) over wood chips, and or in barrels such wood barrels.

For example, such ageing may be carried out for 1 day to 20 years, preferably for 1 month to 10 years, more preferably for 3 years to 8 years.

It is appreciated that the present process is preferably for the production of a spirit. Preferably, the spirit is selected from the group comprising whiskey, vodka, corn-brand, brandy, rice spirit and flavoured spirits.

According to another aspect, it is referred to a spirit, preferably the spirit is selected from the group comprising whiskey, vodka, corn-brand, brandy, rice spirit and flavoured spirits, prepared by the process for the production of ethanol and/or fermentation by-products from starch-containing biomass as defined herein.

Thus, the spirit, preferably spirit selected from the group comprising whiskey, vodka, corn-brand, brandy, rice spirit and flavoured spirits, is prepared by the process for the production of ethanol and/or fermentation by-products from starch-containing biomass comprising the steps of
a) providing a starch-containing biomass,
b) providing yeast,
c) preparing a fermentation mixture by mixing the starch-containing biomass of step a) with water,
d) mechanical comminution of the starch-containing biomass in the fermentation mixture of step c) at a temperature of at most 45°C,
e) adding yeast before and/or during and/or after mechanical comminution step d),
f) incubating the fermentation mixture obtained in comminution step d),
g) distillation of the fermentation mixture obtained in step f) for purifying the prepared ethanol and/or fermentation by-products.

With respect to the definition of the process for the production of ethanol and/or fermentation by-products from starch-containing biomass and preferred embodiments thereof, reference is made to the statements provided above when discussing the technical details of the process of the present invention.

The scope and interest of the invention will be better understood based on the following examples which are intended to illustrate certain embodiments of the present invention and are non-limitative.

### Examples

Different approaches were carried out at 50 L pilot scale. The aim of this was to minimize inaccuracies such as measurement and dosing errors and additionally to produce enough raw spirit to evaluate the sensory characteristics. As a grain-based spirit may only be called whisky in the EU after a storage period of at least 3 years, during which time the aroma changes considerably, a part of the distillate was subjected to a quick maturation with wood chips. The inventive process was compared with two conventional processes, which are predominantly used in Scotland and the USA, respectively. Besides alcohol yield and aroma, the process duration, energy input and processability were evaluated comparatively.

### 1. Materials & Methods

### 1.1. Materials

For each batch, 12.5 kg of classic German Pilsener brewing malt from the IREKS malting plant was used.

As mash water, 37.5 L cold Freising tap water of about 13°C was used.

120 L HDPE lidded barrels were used as fermentation vessels.

The two hot mashes were produced on a "Kleinbrauerei KB50" machine from Alfred Gruber GmbH.

The malt was ground for the hot preparations with a 2-roll mill with a grinding gap of 0.6 mm.

For the inventive process, the mash was ground with a Miccra GmbH rod disperser (8kW) with DS-40/PF SMIR tool.

The enzymes from NovoZymes as set out in Table 1 were stored in the refrigerator and dosed according to the manufacturer's instructions for the "full mash" and "inventive" preparations:

**Table 1**

| **Product name** | **Dosage per 12.5 kg raw material** |
|---|---|
| Attenuzyme Pro | 12.5 ml |
| Ultraflow Max | 3.1 ml |
| Termamyl SC DS | 3.1 ml |
| Ceremix Flex | 25 ml |

All mashes were fermented with the distiller's yeast "Pinnacle MG+" at a dosage of 1g/L.

The distillation took place in a 65 L bladder of the Carl GmbH containing three rectification trays.

Medium toasted American oak chips were used for the quick aging.

### 1.2. Methods

### 1.2.1. Lautering wort

The lautering wort was mashed in at 55°C for 10 min, then heated to 65°C in 10 min. A 60 min saccharification rest followed at this temperature. The mash was then heated to 78°C followed by lautering. The lautering wort was cooled to a pitching temperature of 25°C, aerated and the yeast added.

The process, including crushing, heating the mash water, cooling and cleaning, took about 4 h.

### 1.2.2. Full mash

The full mash was produced according to the process typical for American grain whiskey. For this, the raw material was first gelatinized hot and then gradually cooled with the addition of enzymes.

It was mashed in at 82°C and then the temperature was maintained for 30 min after addition of the enzyme Termamyl. The mash was then cooled to 60°C and the enzymes Ultraflow Max, Attenuzyme Pro, Ceremix Flex were added. After a 30 min rest, the mash including grain components was cooled to 25°C, aerated and the yeast was added.

The process, including crushing, heating the mash water, cooling and cleaning, took about 4 h.

### 1.2.3. Inventive process

For the inventive process, 37.5 L of cold tap water was added to 12.5 kg of unmilled barley malt in the fermentation barrel. The barrel was then positioned under the disperser and dispersed for 5 min at 8000 rpm. During this process, the mixture warmed only slightly (< 25°C). Subsequently, the enzymes and the yeast were added.

The process took a total of 20 minutes.

### 1.2.4. Fermentation

Industry standard amounts of antifoam were added. The fermentation took place without pressure at a controlled room temperature of 30°C. In all preparations, fermentation was completely finished after 5 days.

### 1.2.5. Distillation

The fermented mashes were heated in the bladded and distilled over three trays under automatic pre- and post-run separation. Pre- and post-run were discarded, respectively. After one week, the raw spirits were diluted from 86 vol.-% to barrel (65 vol.-%) or potable (40 vol.-%) strength. The 40 vol.-% samples were bottled, the 65 vol.-% samples were subjected to rapid maturation.

### 1.2.6. Rapid maturation

For the rapid maturation, roasted oak chips were added into the glass balloon to the distillate with 65 vol.-%. There they have been matured for 6 months to absorb aroma and color of the wood and round off in taste.

### 2. Results

### 2.1. Efficiency

The results for the alcohol yield, energy consumption and water consumption for the lautering wort, the full mash and the inventive process are described in the following Tables 2, 3 and 4.

**Table 2: alcohol yield**

| **Fraction** | **Lautering wort** | **Full mash** | **Inventive process** |
|---|---|---|---|
| Heads | 360 ml | 370 ml | 370 ml |
| Heart (87%) | 3460 ml | 4250 ml | 4900 ml |
| Tails (up to 20 vol.-%) | 960 ml | 1100 ml | 900 ml |
| litres of pure alcohol / tonne of raw material | **308** | **363** | **399** |

**Table 3: Energy consumption (estimated values)**

| **Energy source** | **Lautering wort** | **Full mash** | **Inventive process** |
|---|---|---|---|
| Heating mash | 4 kWh | 4 kWh | 0 kWh |
| Agitator | 1 kWh | 1 kWh | 0 kWh |
| Mill | 0.5 kWh | 0.5 kWh | 0 kWh |
| Disperser | 0 kWh | 0 kWh | 0.7 kWh |
| Total | **5.5 kWh** | **5.5 kWh** | **0.7 kWh** |

**Table 4: Water consumption**

| **Water demand** | **Lautering wort** | **Full mash** | **Inventive process** |
|---|---|---|---|
| Mash water | 37.5 L | 37.5 L | 37.5 L |
| Cooling water | 90 L | 90 L | 0 L |
| Cleaning water | 20 L | 20 L | 5 L |
| Total | **147.5** L | **147.5** L | **42.5** L |

From the results obtained, it can be gathered that the inventive process is clearly superior to the classic hot mashing with regard to energy and water consumption and at the same time achieved 9% and 23% higher alcohol yields. For the relevant middle run, the yields were even 13% and 29% higher.

### 2.2. Sensory evalutation

The sensory characteristics play a major role in a high-priced drink like whiskey. The aroma of the raw spirit is only conditionally comparable with the aroma of the finished whiskey due to the strong change over the storage time. The raw spirits were tasted by sensory experts at the TUM Chair of Brewing and Beverage Technology. This showed that the three approaches are sensory distinguishable, but no clear preference is recognizable.

## Claims

1. Process for the production of ethanol and/or fermentation by-products from a starch-containing biomass, comprising the steps of
a) providing a starch-containing biomass,
b) providing yeast,
c) preparing a fermentation mixture by mixing the starch-containing biomass of step a) with water,
d) mechanical comminution of the starch-containing biomass in the fermentation mixture of step c) at a temperature of at most 45°C,
e) adding yeast before and/or during and/or after mechanical comminution step d),
f) incubating the fermentation mixture obtained in comminution step d),
g) distillation of the fermentation mixture obtained in step f) for purifying the prepared ethanol and/or fermentation by-products.

2. The process according to claim 1, wherein the starch-containing biomass is selected from non-malted grain, non-milled grain, non-malted non-milled grain, non-milled malted grain and mixtures thereof or the starch-containing biomass is selected from the group comprising non-malted grain, non-milled grain, non-malted non-milled grain, non-milled malted grain and mixtures thereof in admixture with milled grain or milled malted grain.

3. The process according to claim 1 or 2, wherein the starch-containing biomass is selected from the group comprising barley, rye, wheat, corn, rice, potatoes, oats, buckwheat, sorghum, millet, einkorn, emmer, triticale and mixtures thereof.

4. The process according to any one of claims 1 to 3, wherein the weight ratio of starch-containing biomass to water in step c) ranges from 1:0.3 to 1:5, preferably from 1:0.5 to 1:4, more preferably from 1:1 to 1:4 and most preferably from 1:2 to 1:3.5.

5. The process according to any one of claims 1 to 4, wherein the water in step c) is tap water and/or pre-treated water.

6. The process according to any one of claims 1 to 5, wherein the water in step c) has a temperature ranging from 1 to 25°C, preferably from 10 to 25°C.

7. The process according to any one of claims 1 to 6, wherein the mechanical comminution of step d) is carried out by applying shear force, impact force, frictional force or cutting force.

8. The process according to any one of claims 1 to 7, wherein mechanical comminution step d) is carried out by using a dispersing device.

9. The process according to any one of claims 1 to 8, wherein in step e) the yeast is added after step d), preferably the yeast is added before saccharification of a maximum of 20% of the starch in the fermentation mixture has taken place.

10. The process according to any one of claims 2 to 9, wherein the process further comprises a step e1) of adding starch- and/or sugar-hydrolyzing enzymes and/or enzymes for liquefaction during any one of steps c) to f).

11. The process according to any one of claims 1 to 10, wherein incubating step f) is carried out at 10 to 45°C, preferably at 28 to 45°C, e.g. at about 30°C.

12. The process according to any one of claims 1 to 11, wherein incubating step f) is carried out for 24 to 240 hours, preferably for 48 to 96 hours.

13. The process according to any one of claims 1 to 12, wherein the process further comprises a step h) of aging the ethanol and/or fermentation by-products obtained in distillation step g).

14. The process according to any one of claims 1 to 13, wherein the process is for the production of a spirit, preferably the spirit is selected from the group comprising whiskey, vodka, corn-brand, brandy, rice spirit and flavoured spirits.

15. Spirit, preferably the spirit is selected from the group comprising whiskey, vodka, corn-brand, brandy, rice spirit and flavoured spirits, prepared by the process for the production of ethanol and/or fermentation by-products from starch-containing biomass according to any one of claims 1 to 14.
